Europäisches Patentamt

European Patent Office

Office européen des brevets

(11) Publication number: **0 276 561**
**A1**

(12) **EUROPEAN PATENT APPLICATION**

(21) Application number: **87311135.5**

(22) Date of filing: **17.12.87**

(51) Int. Cl.⁴: **A61L 15/03 , A61K 9/70**

Claims for the following Contracting States: ES + GR..

(30) Priority: **24.12.86 JP 306392/86**

(43) Date of publication of application:
**03.08.88 Bulletin 88/31**

(84) Designated Contracting States:
**AT BE CH DE ES FR GB GR IT LI LU NL SE**

(71) Applicant: **PFIZER INC.**
**235 East 42nd Street**
**New York, N.Y. 10017(US)**

(72) Inventor: **Maeda, Minoru**
**918, Hamakurosaki**
**Toyama-shi(JP)**
Inventor: **Nakamura, Takeshi**
**405, Kenei-jutaku 2 1384-2, Ebara Shimmachi**
**Toyama-shi(JP)**

(74) Representative: **Wood, David John et al**
**Pfizer Limited Ramsgate Road**
**Sandwich Kent CT13 9NJ(GB)**

(54) **Pharmaceutical tape compositions.**

(57) Topical anti-inflammatory/analgesic pharmaceutical compositions for use in tape form which contain piroxicam as the essential active ingredient therein are disclosed. These compositions include the active ingredient in an aqueous dispersion with or without an organic solvent in an alkaline agent in conjunction with an aqueous adhesive base, said compositions having a pH value of from about 4.0 to about 9.0. These novel formulations in tape form are characterized by excellent applicability on the skin, good skin permeability and good stability. A preferred embodiment involves an acrylic acid ester resin emulsion as the aqueous adhesive base, an alkanolamine or ammonium hydroxide as the alkaline agent and benzyl alcohol, propylene glycol or crotamiton as the solubilizing agent.

EP 0 276 561 A1

## PHARMACEUTICAL TAPE COMPOSITIONS

This invention relates to new and useful anti-inflammatory/analgesic pharmaceutical compositions for use in tape form, which contain piroxicam as the essential active ingredient therein. More particularly, it is concerned with certain novel topical anti-inflammatory/analgesic pharmaceutical compositions for external use in tape form, which comprise piroxicam in an aqueous dispersion with or without an organic solvent in an alkaline agent in conjunction with an aqueous adhesive base.

Piroxicam, i.e., 4-hydroxy-2-methyl-N-(2-pyridinyl)-2H-1,2-benzothiazine-3-carboxamide 1,1-dioxide, is a known non-steroidal anti-inflammatory agent described and claimed by J.G. Lombardino in U.S. Patent No. 3,591,584. It possesses excellent anti-inflammatory and analgesic properties. However, non-steroidol anti-inflammatory agents, in general, all exhibit various side effects, including gastrointestinal disorders. Accordingly, when inflammatory symptoms become less severe, it is often desirable to apply piroxicam topically. Additionally, topical administration is also the most suitable way of applying piroxicam to those patients who suffer from localized lesions.

An ointment or a cream is usually a most convenient form for topical administration. However, in the case of piroxicam containing products, the quantity to be applied is often uncertain and the over-all formulation is sticky and troublesome to apply. Additionally, the sticky product will often adhere to clothing after it has been applied with a resultant loss of active ingredient.

Accordingly, an object of the present invention is to provide a new and more suitable composition for the topical administration of piroxicam. Another object of the invention is to increase the absorbability of piroxicam through the skin.

In this connection, it is to be noted that in an ordinary poultice incorporating piroxicam with an aqueous adhesive base of 20-80% water content, there is not much release of piroxicam from the dispersing agent, so that it is rather difficult to attain the blood concentration levels of the active ingredient that are necessary in order to achieve the desired pharmacological effect. Moreover, piroxicam is sparingly soluble in water and most common organic solvents. While several organic solvents are known in which piroxicam is very soluble, these can not be used for the present applications to the skin in view of their volatility. Additionally, the use of suspensions or micropowders for administering piroxicam topically to the skin is rather limited, since these formulations all have the drawback of not allowing sufficient piroxicam drug absorption to occur through the skin in order to achieve the desired pharmacological effect.

In U.S. Patent No. 4,678,666 to S. Nozawa et al., there is described and claimed a piroxicam-containing product in gel ointment form which is designed to overcome some of the above-mentioned drawbacks.

In accordance with the present invention, the above objects have now been achieved and the aforementioned disadvantages largely overcome by the development of a novel anti-inflammatory/analgesic pharmaceutical composition for external use in tape form which contains piroxicam as the essential active ingredient therein. In this way, the drug is rapidly absorbed through the skin and the pharmaceutical effect of the piroxicam medicament is sustained over a long period of time.

More specifically, there is now provided for the first time a topical anti-inflammatory/analgesic pharmaceutical composition for use in tape form, which comprises in an aqueous dispersion with or without an organic solvent a therapeutically-effective anti-inflammatory amount of piroxicam; an alkaline agent, preferably in conjunction with an organic solubilizing agent; and an aqueous adhesive base in sufficient amount to total 100%, said composition having a pH range of from about 4.0 to about 9.0. These novel formulations, when used in tape form, are characterized by excellent applicability on the skin, good skin permeability and good stability.

Also encompassed by this invention is the tape product obtained by coating the above-described topical anti-inflammatory/analgesic pharmaceutical composition as a base layer onto a plastic film or nonwoven tissue support. This is achieved by either coating the composition (base layer) onto a peel-off liner, drying and then stretching over a polyvinyl chloride (PVC) film support or by first coating the base layer onto a PVC film support, followed by drying and then stretching a fresh peel-off liner over the thusly-treated support.

In accordance with a more detailed consideration of the invention, the aqueous adhesive base must be one that has good adhesive properties in the dry state. Suitable adhesive bases for these purposes include (1) acrylic resins that are polymers or copolymers of acrylic acid esters, e.g., copolymers of methyl acrylate with either 2-ethylhexyl acrylate or with itaconic acid; (2) elastic bases like natural rubber or copolymers of styrene and butadiene, and synthetic rubbers such as silicon rubber; (3) resins such as petroleum resin, rosin, hydrogenated rosins and ester gums; and (4) emollients of higher esters such as polybutene, liquid paraffin and isopropyl myristate. Preferred aqueous adhesive bases include acrylic resins in the form of an

2

emulsion, particularly when said acrylic resin is a copolymer of an acrylic acid ester as previously described. In practice, the aqueous adhesive base always constitutes the major portion of the topical anti-inflammatory/analgesic pharmaceutical composition of the present invention and is present in sufficient amount to total 100% by weight of said composition after all the other ingredients have been added.

As regards the piroxicam component of the topical anti-inflammatory/analgesic pharmaceutical composition of this invention, it is only necessary that piroxicam be present at a concentration level that constitutes an effective anti-inflammatory amount, but there is otherwise no restriction on the amount of piroxicam in the composition. The preferred concentration level generally ranges from about 0.1% to about 2.0% by weight of the total composition. Amounts below the lower limit usually afford less than a satisfactory anti-inflammatory effect, while the use of concentration levels above the upper limit is not recommended for economic reasons.

The absorption of piroxicam through the skin can be greatly enhanced by mixing the piroxicam present in the novel compositions of this invention with an alkaline agent contained in the aqueous adhesive base. Suitable alkaline agents for these purposes include ammonia water, i.e., ammonium hydroxide, organic amines like the lower alkanolamines, basic amino acids such as L-arginine and L-lysine, as well as alkali metal hydroxides like sodium hydroxide and potassium hydroxide, etc. The preferred alkaline agents for these purposes are the lower alkanolamines and ammonium hydroxide. Preferred lower alkanolamines include monoalkanolamines having from one to four carbon atoms, dialkanolamines having from two to eight carbon atoms and trialkanolamines having from three to eight carbon atoms. Specific examples of such agents include monoalkanolamines like monoethanolamine, diakanolamines such as di-isopropanolamine and trialkanolamines such as triethanolamine, etc. In general, the amount of alkanolamine present in the final pharmaceutical composition of the invention conveniently ranges anywhere from between about 0.5 to about 10 parts by weight of alkanolamine per one part by weight of piroxicam in order to achieve the desired pharmaceutical effects.

In accordance with a preferred embodiment of the present invention, the mixture obtained after the alkaline agent has already been added can thereafter be further dissolved and dispersed in a solubilizing agent, if so desired, such as a pharmaceutically-acceptable polar protic or aprotic organic solvent. Suitable polar protic organic solvents (or solubilizing agents) for these purposes include benzyl alcohol, lower alkylene glycols, glycerine, ethylene glycol monoethyl ether and polyethylene glycol, while suitable aprotic organic solvents include crotamition (i.e., N-ethyl-o-crotonotoluidine or crotonyl-N-ethyl-o-toluidine) and peppermint oil. The preferred solvents (or solubilizing agents) for these purposes are benzyl alcohol, the lower alkylene glycols and crotamiton. Preferred lower alkylene glycols include those having from two to six carbon atoms such as ethylene glycol, propylene glycol and butylene glycol, etc. In general, the amount of said solvent present in the novel pharmaceutical composition of this invention conveniently ranges from a solubilizing amount up to about 20 parts by weight of solubilizing agent per one part by weight of piroxicam. A preferred lower limit for the solubilizing agent would be about two parts by weight per one part by weight of piroxicam.

In addition to the above-mentioned ingredients, various other ingredients can also be incorporated into the topical anti-inflammatory/analgesic pharmaceutical compositions of this invention to improve their therapeutic efficacy and stability, etc. These include surfactants such as polyoxyethylene sorbitan monostearate and suitable skin permeating agents such as di-isopropyl adipate and diethyl sebecate, etc., which are well-known to those skilled in the art.

The desired pH range for the novel topical anti-inflammatory/analgesic pharmaceutical compositions of this invention is generally one that is from about 4.0 to about 9.0. In this way, any irritant effect on the skin is avoided and the chemical stability of piroxicam is maintained in a facile manner.

In carrying out the process of this invention, piroxicam is first preferably dissolved in an aqueous solution of the alkaline agent, followed by the addition of the solubilizing agent thereto (if so desired) and the stirred mixture thereafter blended into the aqueous adhesive base under conditions of constant agitation to form a homogeneous pharmaceutical composition suitable for use in tape form. The latter step is preferably accomplished by coating the final product onto a peel-off liner, followed by drying and stretching over a polyvinyl chloride (PVC) film as previously described.

The topical anti-inflammatory/analgesic pharmaceutical compositions of this invention so-produced and the tape products resulting therefrom possess a number of distinct advantages, as previously indicated, viz., (1) the tape is very easy to use and apply; (2) the final product does not stick to clothing; (3) it is easy to control the quantity of medicament to be applied; (4) the efficacy of the drug is sustained over a long period of time; (5) good skin permeability of the active ingredient is rapidly achieved; and (6) the required blood concentration levels for the desired pharmacological effect are readily obtained.

In summary, therefore, a preferred pharmaceutical composition of the present invention involves a topical anti-inflammatory/analgesic composition for use in tape form, comprising in an aqueous dispersion with or without an organic solvent from about 0.1% to about 2.0% by weight of piroxicam; from about 0.5 to about 10 parts by weight an alkaline agent per one part by weight of piroxicam; from about zero to about 20 parts by weight of a solubilizing agent per one part by weight of piroxicam, wherein said solubilizing agent is a pharmaceutically-acceptable polar protic or aprotic organic solvent; and an aqueous adhesive base in sufficient amount to total 100%, said composition having a pH range of from about 4.0 to about 9.0.

A particularly preferred composition in this connection involves a topical anti-inflammatory/analgesic pharmaceutical composition for use in tape form, comprising in an aqueous dispersion from about 0.1% to about 2.0% by weight of piroxicam; from about 0.5 to about 10 parts by weight of an alkaline agent per one part by weight of piroxicam, wherein said alkaline agent is a lower alkanolamine or ammonium hydroxide; from up to about 20 parts by weight of a solubilizing agent per one part by weight of piroxicam, wherein said solubilizing agent is a pharmaceutically-acceptable polar protic or aprotic organic solvent; and an acrylic acid ester copolymer resin in the form of an emulsion as an aqueous adhesive base in sufficient amount to total 100%, said composition having a pH range of from about 4.0 to about 9.0.

EXAMPLE 1

A topical anti-inflammatory/analgesic pharmaceutical composition for use in tape form was prepared by homogeneously blending the following ingredients together in the proportions by weight indicated below:

```
Piroxicam.....................................0.5 g.
Di-isopropanolamine...........................1.0 g.
Propylene glycol..............................2.0 g.
Di-isopropyl adipate..........................1.0 g.
Acrylic resin emulsion A[1]..................159.2 g.
Total                                        163.7 g.
(based on 100.0 g. of solids)
[1]Emulsion of methyl acrylate and 2-ethylhexyl acrylate
copolymer resin (solids 60%)
```

The mixture so obtained was then coated onto a peel-off liner, dried and the resulting material stretched over a polyvinyl chloride (PVC) film to afford the final product in tape form.

EXAMPLE 2

A topical anti-inflammatory/analgesic pharmaceutical composition for use in tape form was prepared by homogeneously blending the following ingredients together in the proportions by weight indicated below:

```
Piroxicam ...............................0.5 g.
Di-isopropanolamine......................1.0 g.
Crotamiton...............................1.0 g.
Di-isopropyl adipate.....................1.0 g.
Acrylic resin emulsion A[1].............160.8 g.
Total                                   164.3 g.
```
(based on 100.0 g. of solids)

[1]Emulsion of methyl methacrylate and 2-ethylhexyl acrylate copolymer resin (solids 60%)

The mixture so obtained was then coated onto a peel-off liner, dried and the resulting material stretched over a polyvinyl chloride (PVC) film to afford the final product in tape form.

EXAMPLE 3

A topical anti-inflammatory/analgesic pharmaceutical composition for use in tape form was prepared by homogeneously blending the following ingredients together in the proportions by weight indicated below:

```
Piroxicam................................0.5 g.
Strong ammonia water.....................1.0 g.
Benzyl alcohol...........................1.0 g.
Di-isopropyl adipate.....................1.0 g.
Polysorbate 60[2]........................1.0 g.
Acrylic resin emulsion B[3].............166.4 g.
Total                                   170.9 g.
```
(based on 100.0 g. of solids)

[2]Polyoxyethylene sorbitan monostearate

[3]Emulsion of methyl acrylate and itaconic acid copolymer resin (solids 60%)

The mixture so obtained was then coated onto a peel-off liner, dried and the resulting material stretched over a polyvinyl chloride (PVC) film to afford the final product in tape form.

EXAMPLE 4

A topical anti-inflammatory/analgesic pharmaceutical composition for use in tape form was prepared by homogeneously blending the following ingredients together in the proportions by weight indicated below:

```
Piroxicam...................................0.5 g.
Strong ammonia water.......................1.0 g.
Adhesive C⁴................................360.0 g.
Polybutene.................................4.0 g.
Liquid paraffin...........................23.0 g.
Total                                     389.0 g.
(based on 100.0 g. of solids)

⁴Natural rubber as solids..................32.0 g.
 Synthetic rubber as solids.................8.0 g.
 Ester gum HP as solids....................32.0 g.
    (pentaerythritol ester of hydrogenated rosin)
```

The mixture so obtained was then coated onto a pell-off liner, dried and the resulting material stretched over a polyvinyl chloride (PVC) film to afford the final product in tape form.

EXAMPLE 5

A topical anti-inflammatory/analgesic pharmaceutical composition for use in tape form was prepared by homogeneously blending the following ingredients together in the proportions by weight indicated below:

```
Piroxicam...................................0.3 g.
Triethanolamine............................1.6 g.
Silicon rubber............................58.0 g.
Hydrogenated rosin........................28.0 g.
Light liquid paraffin......................5.2 g.
Stearic Acid...............................6.9 g.
Water.....................................66.0 g.
Total                                     166.0 g.
(based on 100.0 g. of solids)
```

The mixture so obtained was then coated onto a peel-off liner, dried and the resulting material stretched over a polyvinyl chloride (PVC) film to afford the final product in tape form.

EXAMPLE 6

6

Skin Absorption Test

The abdominal hair on a male Wistar rat weighing approximately 200 g. was shaved with an electric Varican, and the tape product of Example 1 was applied to the shaved area on the following day. After a certain period of time had elaspsed, viz., at 6, 9, 12 and 16 hour intervals after topical administration, blood was sampled from the main abdominal artery and the concentration of active ingredient (i.e., piroxicam) in the serum was measured by means of high pressure liquid chromatography (HPLC). The results obtained in this manner are shown below in Table 1, together with the results obtained with a conventional poultice base having the following composition:

```
Piroxicam..................................0.5 g.
Di-isopropanolamine........................2.0 g.
Peppermint Oil.............................1.0 g.
Propylene glycol..........................15.0 g.
Polyvinyl alcohol..........................2.0 g.
Sodium polyacrylate........................5.0 g.
Gelatine...................................7.0 g.
Sodium carboxymethyl cellulose.............3.0 g.
Kaolin....................................15.0 g.
Zinc oxide.................................3.0 g.
Concentrated glycerin.....................15.0 g.
Distilled water...........................31.5 g.
Total                                    100.0 g.
```

## TABLE 1

### Concentration in Blood

| Time | $n^1$ | Tape of Example 1 ng/ml (means $\pm$ SE$^2$) | $n$ | Conventional poultice composition ng/ml (means $\pm$ SE) |
|---|---|---|---|---|
| 6 hrs. | 5 | 1862.6$\pm$325.4 | 17 | 122.3$\pm$13.2 |
| 9 hrs. | 5 | 917.2$\pm$170.0 | 12 | 102.9$\pm$10.3 |
| 12 hrs. | 10 | 1257.5$\pm$272.0 | 16 | 149.5$\pm$14.9 |
| 16 hrs. | 5 | 906.8$\pm$213.9 | 18 | 154.3$\pm$20.9 |

[1] n = number of tests

[2] SE = standard error

EXAMPLE 7

Anti-inflammatory Test

This test is based on the suppression of a red spot produced on the skin by exposure to ultraviolet (UV) light. In order to carry out this test, five male mormots (each weighing approximately 250-300 g.) were used. The abdominal hair was shaved, and the piroxicam tape product of Example 1 was applied to the shaved abdominal area on the following day, with the tape then being covered with an elastic protective bandage. After a period of one hour, the abdominal area was exposed to UV radiation by conventional means. The development of a red spot was then evaluated at two and five hour intervals after exposure to the light, according to a three-part scale defined in the following manner, viz., 1.0 point represents an irradiated (reddened) area having a well-defined boundary; 0.5 point indicates a red spot with an ill-defined boundary; and 0 point means practically no red spot is visible. The results obtained in this manner are summarized below in the following table (Table 2), where a comparison is made with the control (no tape):

## TABLE 2
### Suppression of Red Spot produced by UV Light

| | Rat No. | Score (sum of points) | |
| --- | --- | --- | --- |
| | | 2 hours | 5 hours |
| Tape of | 1 | 0 | 0 |
| Example | 2 | 0 | 0 |
| 1 | 3 | 0 | 0 |
| | 4 | 0 | 0 |
| | 5 | 0 | 0 |
| Control | 1 | 3 | 3 |
| (no tape) | 2 | 3 | 3 |
| | 3 | 3 | 3 |
| | 4 | 3 | 3 |
| | 5 | 3 | 3 |

**Claims**

1. A topical anti-inflammatory/analgesic pharmaceutical composition for use in tape form, comprising in an aqueous dispersion with or without an organic solvent from 0.1% to 2.0% by weight of piroxicam; from 0.5 to 10 parts by weight of an alkaline agent per one part by weight of piroxicam, wherein said alkaline agent is a lower alkanolamine or ammonium hydroxide; from zero to 20 parts by weight of a solublizing agent per one part by weight of piroxicam, wherein said solubilizing agent is a pharmaceutically-acceptable polar protic or aprotic organic solvent; and an aqueous adhesive base in sufficient amount to total 100%, said composition having a pH range of from 4.0 to 9.0.

2. A composition as claimed in claim 1 wherein said lower alkanolamine is a monoalkanolamine having from one to four carbon atoms, a dialkanolamine having from two to eight carbon atoms or a trialkanolamine having from three to twelve carbon atoms.

3. A composition as claimed in claim 2 wherein said dialkanolamine is di-isopropanolamine and said trialkanolamine is triethanolamine.

4. A composition as claimed in any one of the preceding claims wherein said polar protic organic solvent is benzyl alcohol or a lower alkylene glycol having from two to six carbon atoms.

5. A composition as claimed in claim 4 wherein said lower alkylene glycol is propylene glycol.

8

6. A composition as claimed in any one of claims 1 to 3 wherein said aprotic organic solvent is crotamiton.

7. A composition as claimed in any one of the preceding claims wherein said aqueous adhesive base is an acrylic resin in the form of an emulsion.

8. A composition as claimed in claim 7 wherein said acrylic resin is a polymer or copolymer of an acrylic acid ester.

9. A composition as claimed in claim 8 wherein said acrylic acid ester copolymer is a copolymer of methyl acrylate and 2-ethylhexyl acrylate.

10. A composition as claimed in claim 8 wherein said acrylic acid ester copolymer is a copolymer of methyl acrylate and itaconic acid.

11. A tape comprising a support coated with a composition as claimed in any one of the preceding claims.

CLAIMS for the following Contracting States: ES and GR

1. A process for preparing a topical anti-inflammatory/analgesic pharmaceutical composition, for use in tape form, in an aqueous dispersion with or without an organic solvent, characterized by the step of blending piroxicam into an aqueous adhesive base containing an alkaline agent and optionally an organic solubilizing agent, wherein said alkaline agent is a lower alkanolamine or ammonium hydroxide and said solubilizing agent is a pharmaceutically-acceptable polar protic or aprotic organic solvent, and thereafter subjecting the resulting aqueous organic mixture to constant agitation to form a homogeneous pharmaceutical composition suitable for use in tape form; the amounts of the ingredients being such as to provide the topical pharmaceutical composition so formed with from 0.1% to 2.0% by weight of piroxicam, from 0.5 to 10 parts by weight alkaline agent per one part by weight of piroxicam, from zero to 20 parts by weight of solubilizing agent per one part by weight of piroxicam, and an aqueous adhesive base in sufficient amount to total 100%, said composition having a pH in the range of from 4.0 to 9.0.

2. A process as claimed in claim 1, characterized by the fact that said lower alkanolamine is a monoalkanolamine having from one to four carbon atoms, a dialkanolamine having from two to eight carbon atoms or a trialkanolamine having from three to twelve carbon atoms.

3. A process as claimed in claim 12, characterized by the fact that said dialkanolamine is diisopropanolamine and said trialkanolamine is triethanolamine.

4. A process as claimed in any one of claims 1 to 3, characterized by the fact that said polar protic organic solvent is benzyl alcohol or a lower alkylene glycol having from two to six carbon atoms.

5. A process as claimed in claim 4, characterized by the fact that said lower alkylene glycol is propylene glycol.

6. A process as claimed in any one of claims 1 to 3, characterized by the fact that said aprotic organic solvent is crotamiton.

7. A process as claimed in any one of the preceding claims, characterized by the fact that said aqueous adhesive base is an acrylic resin in the form of an emulsion.

8. A process as claimed in claim 7, characterized by the fact that said acrylic resin is a polymer or copolymer of an acrylic acid ester.

9. A process as claimed in claim 8, characterized by the fact that said acrylic acid ester copolymer is a copolymer of methyl acrylate and 2-ethylhexyl acrylate.

10. A process as claimed in claim 8, characterized by the fact that said acrylic acid ester copolymer is a copolymer of methyl acrylate and itaconic acid.

11. A process according to any one of the preceding claims, characterized by the additional step of coating the composition onto a suitable carrier so as to form a tape.

12. A process according to claim 11, characterized by coating the composition onto a peel-off liner, drying and then stretching over a polyvinyl chloride film support.

13. A tape comprising a support coated with a topical antiinflammatory/analgesic composition, said composition comprising in an aqueous dispersion with or without an organic solvent from 0.1% to 2.0% by weight of piroxicam; from 0.5 to 10 parts by weight of an alkaline agent per one part by weight of piroxicam, wherein said alkaline agent is a lower alkanolamine or ammonium hydroxide; from zero to 20 parts by weight of a solublizing agent per one part by weight of piroxicam, wherein said solubilizing agent is a pharmaceutically-acceptable polar protic or aprotic organic solvent; and an aqueous adhesive base in sufficient amount to total 100%, said composition having a pH range of from 4.0 to 9.0.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,X | EP-A-0 101 178 (PFIZER CORP.) <br> * Page 3, line 20 - page 10, line 17; claims 1-7 * | 1-5 | A 61 L 15/03 <br> A 61 K 9/70 |
| D,Y | | 1-11 | |
| | --- | | |
| Y | EP-A-0 159 168 (TAKEDA CHEMICAL INDUSTRIES) <br> * Page 3, line 14; page 7, line 27 - page 9, line 33; page 14, line 33 - page 15, line 14; claims 1-15 * | 1-11 | |
| | ----- | | |

TECHNICAL FIELDS SEARCHED (Int. Cl.4)

A 61 K
A 61 L
A 61 M

The present search report has been drawn up for all claims

| Place of search | Date of completion of the search | Examiner |
|---|---|---|
| THE HAGUE | 13-04-1988 | TZSCHOPPE,D.A. |

EPO FORM 1503 03.82 (P0401)